# EUROPEAN PATENT APPLICATION

(11) **EP 4 678 662 A1**
(43) Date of publication of application: **14.01.2026**
(21) Application number: 24767115.9
(22) Date of filing: 04.03.2024
(51) Int. Cl.: C07K 16/46, A23K 20/147, A23L 33/18, A61K 39/395, A61P 31/04, A61P 37/04, C12N 1/15, C12N 1/19, C12N 1/21, C12N 5/10, C12N 15/13, C12N 15/62, C12P 21/08

(54) **IMMUNOGLOBULIN**

(30) Priority: 03.03.2023 JP 2023033251
(71) Applicant: The University of Tokyo, Bunkyo-ku, Tokyo 113-8654 (JP)
(72) Inventor: SHINKURA, Reiko, Tokyo 113-8654 (JP)
(74) Representative: Symbiosis IP Limited
(86) International application number: PCT/JP2024/008128
(87) International publication number: WO 2024/185747

(57) **Abstract**

The present disclosure provides an immunoglobulin multimer, a method for producing the same, and an immunoglobulin variant having an ability to form an immunoglobulin multimer. In another aspect, the present disclosure has the effect of providing a method using an immunoglobulin multimer or an immunoglobulin variant having an ability to form an immunoglobulin multimer, and a composition containing the immunoglobulin multimer or the immunoglobulin variant having an ability to form an immunoglobulin multimer.

## Description

### Technical Field

The present disclosure relates to a method for producing an immunoglobulin multimer, an immunoglobulin multimer, an immunoglobulin variant having an ability to form an immunoglobulin multimer, a composition containing the same, and uses thereof.

### Background Art

Immunoglobulins include various isotypes, such as IgG, IgM, IgA, IgD, and IgE, each exhibiting distinct functions in vivo. It is known that immunoglobulins such as IgA and IgM form multimers such as dimers and pentamers, and function in vivo. It is considered that immunoglobulins, when in a multimeric form, exhibit higher biological activity than monomeric immunoglobulins due to the increased number of antigen-binding sites, and that the greater the number of immunoglobulins contained in the multimer, the higher the biological activity.

Various methods for producing immunoglobulin multimers have been studied to date (for example, Patent Literature 1: WO 2021/193553 A1). However, there is still a demand for a method that enables stable and high-quality production of immunoglobulin multimers.

### Citation List

### Patent Literature

Patent Literature 1: WO 2021/193553 A1

### Summary of Invention

### Technical Problem

The present disclosure provides an immunoglobulin multimer, a method for producing the same, and an immunoglobulin variant that can be used in the method. In another aspect, the present disclosure provides a method and a composition using an immunoglobulin multimer and an immunoglobulin variant.

### Solution to Problem

As a result of investigating methods for artificially producing immunoglobulin multimers, the present inventors have unexpectedly found that the use of an immunoglobulin variant having a deletion at the C-terminus of at least one heavy chain enables efficient production of high-quality immunoglobulin multimers. In particular, the present inventors have found that high-quality immunoglobulin multimers can be efficiently obtained by adding a heterologous amino acid sequence to the C-terminus of an immunoglobulin variant having a deletion at the C-terminus of at least one heavy chain, in order to promote multimerization.

Based on the above findings, in one aspect, the present disclosure provides a method for producing an immunoglobulin multimer.

In another aspect, the present disclosure provides an immunoglobulin multimer.

In another aspect, the present disclosure provides an immunoglobulin variant having an ability to form an immunoglobulin multimer.

In another aspect, the present disclosure provides a pharmaceutical composition, a food composition, a feed composition, or a reagent composition containing an immunoglobulin multimer or an immunoglobulin variant.

In another aspect, the present disclosure provides a method for treating or preventing a disease, disorder, or condition using an immunoglobulin multimer or an immunoglobulin variant.

In another aspect, the present disclosure provides a method for testing, examining, or diagnosing using an immunoglobulin multimer or an immunoglobulin variant.

More specifically, the present disclosure provides the following.

### [Item 1]

A method for producing an immunoglobulin multimer, the method comprising:
a step of bringing two or more immunoglobulins into contact with each other, the two or more immunoglobulins containing at least one immunoglobulin variant having a deletion in a C-terminal multimerization-promoting region of at least one heavy chain; and
a step of binding the two or more immunoglobulins.

### [Item 2]

The method according to Item 1, wherein the deletion is a deletion of one or more amino acids in the multimerization-promoting region.

### [Item 3]

The method according to Item 1, wherein a heterologous amino acid sequence that promotes multimerization is added to a C-terminus of an immunoglobulin variant having a deletion in a C-terminal multimerization-promoting region.

### [Item 4]

The method according to Item 1, wherein each of the plurality of immunoglobulins is IgA or IgM.

### [Item 5]

An immunoglobulin multimer produced by the method according to Item 1.

### [Item 6]

An immunoglobulin multimer containing two or more immunoglobulins, wherein at least one of the immunoglobulins constituting the two or more immunoglobulins is an immunoglobulin variant having a deletion in a C-terminal multimerization-promoting region of at least one heavy chain.

### [Item 7]

The immunoglobulin multimer according to Item 5 or 6, wherein the immunoglobulin multimer has a bacterial growth inhibitory activity.

### [Item 8]

The immunoglobulin multimer according to Item 5 or 6, wherein the immunoglobulin multimer has a bacterial agglutination activity.

### [Item 9]

An immunoglobulin variant having a deletion in a C-terminal multimerization-promoting region and having an ability to form an immunoglobulin multimer.

### [Item 10]

A food composition, a feed composition, a pharmaceutical composition, or a reagent composition containing the immunoglobulin multimer according to Item 5 or 6 or the immunoglobulin variant according to Item 9.

### [Item 11]

A nucleic acid containing a nucleic acid sequence encoding the immunoglobulin variant according to Item 9.

### [Item 12]

A cell containing the nucleic acid according to Item 11.

### Advantageous Effects of Invention

The present disclosure provides an immunoglobulin multimer, a method for producing the same, and an immunoglobulin variant having an ability to form an immunoglobulin multimer. In another aspect, the present disclosure has the effect of providing a method using an immunoglobulin multimer or an immunoglobulin variant having an ability to form an immunoglobulin multimer, and a composition containing the immunoglobulin multimer or the immunoglobulin variant having an ability to form an immunoglobulin multimer.

### Brief Description of Drawings

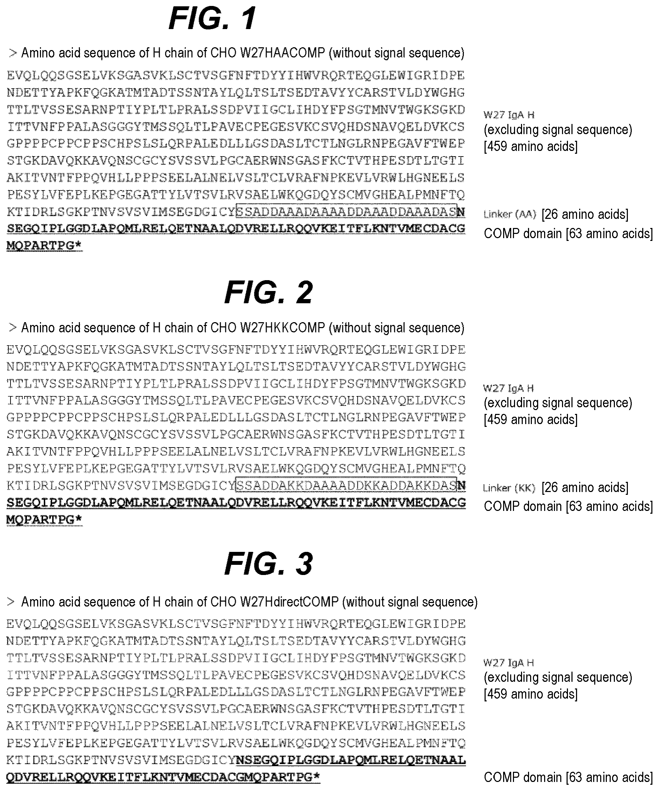

Fig. 1 is a diagram showing the amino acid sequence of the heavy chain of the antibody CHO W27HAACOMP (the signal sequence is not shown).
Fig. 2 is a diagram showing the amino acid sequence of the heavy chain of the antibody CHO W27HKKCOMP (the signal sequence is not shown).
Fig. 3 is a diagram showing the amino acid sequence of the heavy chain of the antibody CHO W27HdirectCOMP (the signal sequence is not shown).
Fig. 4 is a diagram showing the amino acid sequence of the heavy chain of the antibody CHO W27notailCOMP (the signal sequence is not shown).
Fig. 5 is a diagram showing the results of studying the effect of the structure of the C-terminal region of IgA H chain and the structure of the linker on multimer formation.
Fig. 6 is a diagram showing the confirmation of the binding ability of the W27 IgA multimer to E. coli.

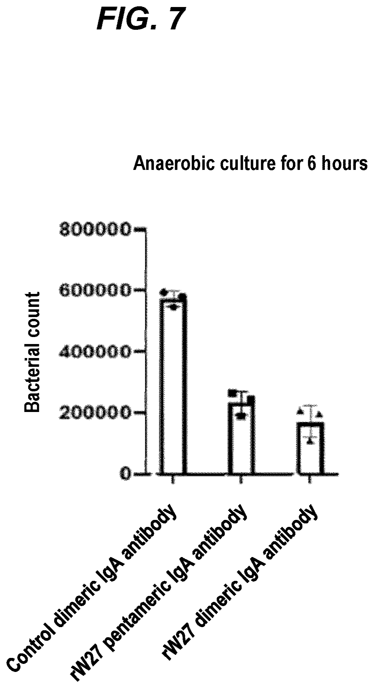

Fig. 7 is a diagram showing the results confirming that the immunoglobulin multimer has an excellent in vitro bacterial growth inhibitory effect.

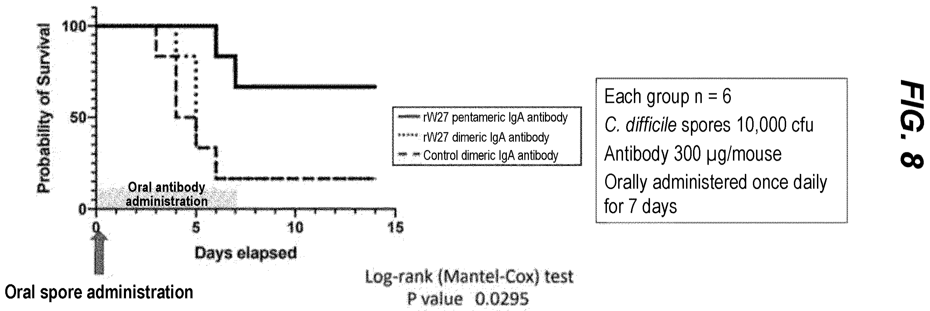

Fig. 8 is a diagram showing the results confirming that the immunoglobulin multimer has an excellent in vivo inhibitory effect on bacteria-associated enteritis.

### Description of Embodiments

The inventors of the present disclosure have attempted to produce immunoglobulin multimers, but it was particularly difficult to efficiently produce immunoglobulin multimers that retained biological activity. As a result of further independent trial and error, the present inventors have unexpectedly found that high-quality immunoglobulin multimers can be efficiently obtained by using an immunoglobulin variant having a deletion in a C-terminal multimerization-promoting region of a heavy chain. In particular, the present inventors have unexpectedly found that high-quality immunoglobulin multimers can be efficiently obtained by adding a heterologous amino acid sequence to the C-terminus of the heavy chain of the immunoglobulin variant to promote multimerization.

### (Definition)

In the present specification, in a case where a plurality of ranges of numerical values are indicated, a range including any combination of a lower limit value and an upper limit value of the plurality of ranges is also meant in the same manner.

Unless defined otherwise, all technical terms and scientific terms used in the present specification have the same meanings as commonly understood by those skilled in the art to which the present disclosure pertains.

In the present specification, a case where the term "about" used in connection with a numerical value x means that the value may vary, for example, within 10%, 9%, 8%, 7%, 6%, 5%, 4%, 3%, 2%, 1%, 0.5%, 0.1%, 0.05%, or 0.01%.

In the present specification, the term "containing" has the same meaning as commonly understood by those skilled in the technical field to which the present disclosure pertains, but includes, for example, "containing" and "consisting of", and specifically means that a composition "containing" A may also contain B as another component, in addition to containing only A.

In the present specification, the "separating" has the same meaning as commonly understood by those skilled in the technical field to which the present disclosure pertains, and means, for example, selectively obtaining certain components from a mixed composition and excluding other components. In the present specification, in a case where an immunoglobulin is separated from a culture medium, "separating" may have the same meaning as "isolating", "purifying", "enriching", and "concentrating".

In the present specification, the "protein" refers to a substance composed of a polypeptide in which amino acids are linked by peptide bonds. In the present specification, a molecular weight of the protein is not particularly limited. Thus, in the present specification, the "protein" also includes the meaning of a polypeptide and a peptide.

In the present specification, the "deletion" has the same meaning as commonly understood by those skilled in the art in the technical field to which the present disclosure pertains, and refers, for example, to a reduction or complete loss of a function or property originally possessed by a target molecule, region, or the like.

In the present specification, the "variant" has the same meaning as commonly understood by those skilled in the art in the technical field to which the present disclosure pertains, and refers, for example, in the case where a subject is a protein, to a variant having one or more substitutions, deletions, or additions in the amino acid sequence. In another aspect, a variant refers to one to which a heterologous molecule is bound.

### (Method for Producing Immunoglobulin Multimer)

In one aspect, the present disclosure provides a method for producing an immunoglobulin multimer. Typically, the method for producing an immunoglobulin multimer of the present disclosure has the following configuration.

The method for producing an immunoglobulin multimer comprising:
a step of bringing two or more immunoglobulins into contact with each other, the two or more immunoglobulins containing at least one immunoglobulin variant having a deletion in a C-terminal multimerization-promoting region of at least one heavy chain; and
a step of binding the two or more immunoglobulins.

The method of the present disclosure may further comprise a step of isolating the immunoglobulin multimer generated by the binding.

In the method of the present disclosure, the environment in which the step of bringing the two or more immunoglobulins into contact with each other is performed is not particularly limited as long as it allows binding of the two or more immunoglobulins. For example, it can be performed in an environment such as within a cell, in a cell suspension, or in vitro in the presence of cellular components. In one aspect, the step of bringing the two or more immunoglobulins into contact with each other can be realized by bringing two or more immunoglobulins into contact with each other within a cell by expressing a nucleic acid encoding the immunoglobulin within the cell.

In the method of the present disclosure, the environment in which the step of binding the two or more immunoglobulins is performed is not particularly limited as long as it allows binding of the two or more immunoglobulins. For example, it can be performed in an environment such as within a cell, in a cell suspension, or in vitro in the presence of cellular components. In one aspect, the step of binding the two or more immunoglobulins can be realized by an interaction between globulin molecules by bringing two or more immunoglobulins into contact with each other within a cell. The modes of binding between the two or more immunoglobulins encompass all forms, and include, for example, covalent bonds, ionic bonds, and hydrophobic bonds. The binding between the two or more immunoglobulins may be mediated by a heterologous molecule bound to the immunoglobulin.

In the method of the present disclosure, the step of isolating the immunoglobulin multimer generated by the binding is not particularly limited, and can be performed using various methods commonly used for protein isolation in the technical field of the present disclosure. For example, techniques such as affinity chromatography, ion exchange chromatography, hydrophobic interaction chromatography, ethanol precipitation, reverse phase HPLC, chromatography on silica or with a cation exchange resin such as DEAE, chromatofocusing, SDS-PAGE, ammonium sulfate precipitation, and gel filtration can be used alone or in combination.

In the method for producing an immunoglobulin multimer of the present disclosure, in the step of isolating the immunoglobulin multimer in the culture medium from the other culture medium components, the produced immunoglobulin multimer can be isolated according to a standard method. In the method for producing an immunoglobulin multimer of the present disclosure, it is not essential to perform cell-disrupting treatments, such as cell lysis, for the isolation and purification of the immunoglobulin multimer. In a preferred aspect, the method for producing an immunoglobulin multimer of the present disclosure does not include a cell-disrupting treatment. In one aspect, the cell used in the method for producing an immunoglobulin multimer of the present disclosure may produce immunoglobulins within a cell. A cell containing an immunoglobulin multimer within a cell can itself be contained as an active ingredient in a composition. For example, when a yeast or a filamentous fungus is used as a cell that produces an immunoglobulin multimer, a cell itself that produces an immunoglobulin multimer can be orally administered. When a cell containing such an immunoglobulin multimer is orally administered, the cell may be disrupted prior to administration, but may not be disrupted.

When a cell is used in the method of the present disclosure, the cell may express two or more immunoglobulin variants having different types of deletion as immunoglobulin variants having a deletion in a C-terminal multimerization-promoting region of at least one heavy chain. In addition, as one aspect, the cell used in the method of the present disclosure may express an immunoglobulin having no deletion in the C-terminal multimerization-promoting region in addition to an immunoglobulin having a deletion in the C-terminal multimerization-promoting region of at least one heavy chain.

In the method of the present disclosure, the type of the produced immunoglobulin multimer is not particularly limited, and can be selected from, for example, IgG, IgA, IgM, IgD, or IgE. In a preferred aspect, the immunoglobulin is IgA or IgM. In a preferred aspect, the obtained immunoglobulin multimer has a physiological activity, for example, a binding activity to an antigen.

The immunoglobulin multimer produced by the method of the present disclosure may contain immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. In a preferred aspect, the immunoglobulin is a human immunoglobulin.

The immunoglobulin produced by the method of the present disclosure may have a wild-type amino acid sequence in the heavy chain or light chain, in addition to the deletion in the C-terminal multimerization-promoting region, or may have a mutation relative to the wild type. The mutation includes addition, deletion, and substitution of an amino acid, and includes shortening of an N-terminus or C-terminus. The properties such as antigen binding ability and stability of the immunoglobulin to be produced can be adjusted by the mutation.

The immunoglobulin multimer produced by the method of the present disclosure can contain, together with an immunoglobulin having a deletion in a C-terminal multimerization-promoting region, an immunoglobulin that does not have the deletion. The immunoglobulin having a deletion in a C-terminal multimerization-promoting region and the immunoglobulin that does not have the deletion may have a wild-type amino acid sequence in the heavy chain or light chain, or may have a mutation relative to the wild type. The mutation includes addition, deletion, and substitution of an amino acid, and includes shortening of an N-terminus or C-terminus. The properties such as antigen binding ability and stability of the immunoglobulin to be produced can be adjusted by the mutation.

In one aspect, the immunoglobulin constituting the immunoglobulin multimer produced by the method of the present disclosure may be a mixture of structures of IgG, IgA, IgM, IgD, or IgE. For example, the immunoglobulin may be IgG, IgA, IgM, IgD, or IgE to which a multimerization-promoting region of IgA or IgM or another region is added by recombinant techniques, or in which a native region is partially replaced by such a region. Alternatively, the immunoglobulin constituting the immunoglobulin multimer produced by the method of the present disclosure may be IgA or IgM in which a portion other than the multimerization-promoting region is replaced with that of another class of globulin. In the present disclosure, when it is stated that the immunoglobulin is IgG, IgA, IgM, IgD, or IgE, this includes IgG, IgA, IgM, IgD, or IgE that contains a part of another type of immunoglobulin and/or is partially replaced with a part of another type of immunoglobulin. When it is stated that the immunoglobulin constituting the immunoglobulin multimer produced by the method of the present disclosure is IgG, IgA, IgM, IgD, or IgE, this includes each of IgG, IgA, IgM, IgD, and IgE that contains a part of another type of immunoglobulin and/or is partially replaced with a part of another type of immunoglobulin.

The immunoglobulin multimer produced by the method of the present disclosure contains two or more immunoglobulins. In a preferred aspect, the immunoglobulin multimer produced by the method of the present disclosure contains 2 to 3, 2 to 4, 2 to 5, 2 to 6, 2 to 7, 2 to 8, or 2 to 9 immunoglobulins.

As the immunoglobulin variant having a deletion in the C-terminal multimerization-promoting region of at least one heavy chain, which is used in the method of the present disclosure, an immunoglobulin variant described in the following (Immunoglobulin Variant Having Deletion in C-Terminal Multimerization-Promoting Region) can be used.

The cell expressing an immunoglobulin variant having a deletion in a C-terminal multimerization-promoting region of at least one heavy chain is not particularly limited and may be appropriately selected by those skilled in the art. In one aspect, it is preferable to use a cell capable of producing various proteins derived from mammals or the like that exhibit functions by forming a higher order structure. As the cell, known cells such as mammalian-derived cells including, for example, COS cells, HEK cells, HeLa cells, and CHO cells, insect-derived cells such as Sf9, and cells such as yeast and filamentous fungi may be appropriately selected, but are not limited thereto.

The cell expressing the immunoglobulin or immunoglobulin variant can be obtained by a standard method using genetic engineering. For example, it can be obtained by introducing a nucleic acid encoding the immunoglobulin or immunoglobulin variant into a cell capable of producing an immunoglobulin.

When introducing a nucleic acid encoding an immunoglobulin or the like into the cell used in the method of the present disclosure, those skilled in the art can adopt a specific method for introducing the nucleic acid based on publicly known information. For example, the nucleic acid may be introduced in the form of a plasmid, an artificial chromosome, or the like, separately from the genome of the host cell, or may be incorporated into the genome of the host cell using genome editing techniques or the like. For the genome modification technique (genome editing technique) used in the method of the present disclosure, various methods commonly used in the technical field of the present disclosure can be used. For example, CRISPR-Cas system, meganucleases (MNs), zinc finger nucleases (ZFN), transcription activation-like effector nucleases (TALENs), and the like can be used. In one aspect, it is preferable to use a modification technique in which a plasmid for genome modification does not remain in the host cell after modification. For example, when a filamentous fungus is used as the host cell, it is possible to obtain a genome-modified cell in which a plasmid for genome modification does not remain in the host cell after modification by using a genome editing plasmid for producing a multi-mutant strain generated through multistage operations disclosed in JP 2018-191551 A for a single-step mutation operation or a multi-step mutation operation.

Various vectors or nucleic acid constructs commonly used in the technical field of the present disclosure can be used as vectors or nucleic acid constructs used in the genome modification operation included in the method for producing a host cell of the present disclosure. For example, a vector containing a selectable marker gene, a cloning site, and a control region (a promoter and a terminator) disclosed in JP 2012-179011 A can be used. In one aspect, in a case where the CRISPR-Cas system is used for the genome modification operation, a vector containing a DNA fragment AMA1 derived from A. nidulans which enables autonomous replication of a plasmid and a DNA fragment designed to highly express Aoace2 gene which significantly deteriorates growth due to high expression, which is disclosed in JP 2018-191551 A, can be suitably used.

Those skilled in the art can appropriately adjust the culture conditions, the collection method, the purification method, and the like of the cells into which the nucleic acid is introduced, depending on the type of cells or the like, and can produce the immunoglobulin multimer according to the present disclosure.

In the step of culturing the cells, conditions for culturing the cells, for example, a composition of a medium, a culture temperature, a medium pH, and shaking conditions can be arbitrarily adjusted and optimized based on techniques known to those skilled in the art. By optimizing these conditions, the amount, yield, quality, and the like of the immunoglobulin can be improved, and an immunoglobulin multimer having desired properties in stability and the like can be produced.

In one aspect, a pH of the medium in which the cells are cultured can be adjusted at any pH of 4 to 11. Preferably, the pH of the medium is selected from a range of 4.0 to 11.0, 4.5 to 10.0, 5.0 to 9.0, 5.5 to 8.5, or 6.0 to 8.0. In one aspect, the pH of the medium can be set within a range of 5.0 or more, 5.5 or more, 6.0 or more, 6.5 or more, 7.0 or more, 7.5 or more, 8.0 or more, 8.5 or more, 9.0 or more, or 9.5 or more, and 11.0 or less, 10.5 or less, 10.0 or less, 9.5 or less, 9.0 or less, 8.5 or less, 8.0 or less, 7.5 or less, 7.0 or less, 6.5 or less, 6.0 or less, 5.5 or less, or 5.0 or less, or any combination of an upper limit and a lower limit thereof.

In the method of the present disclosure, when an in vitro environment containing a cell suspension or a cellular component is used, the cells and the cellular component used for these are not particularly limited, and the method can be performed using various structures used for expression, reaction, and the like of proteins in the technical field of the present disclosure.

The separated immunoglobulin in a multimer form can be confirmed using various methods commonly used in the technical field of the present disclosure. For example, it can be confirmed that the immunoglobulin is a multimer by analyzing the immunoglobulin separated from the culture medium by sodium dodecyl sulfate-polyacrylamide gel electrophoresis (SDS-PAGE) in a non-reduced state.

### (Immunoglobulin Multimer)

In one aspect, the present disclosure provides an immunoglobulin multimer. Typically, the immunoglobulin multimer provided by the present disclosure has the following structure.

An immunoglobulin multimer containing two or more immunoglobulins, in which at least one of the immunoglobulins constituting the two or more immunoglobulins is an immunoglobulin variant having a deletion in a C-terminal multimerization-promoting region of at least one heavy chain.

In one aspect, the immunoglobulin multimer of the present disclosure is produced by the production method described in the (Method for Producing Immunoglobulin Multimer) above.

Accordingly, the immunoglobulin multimer of the present disclosure is an immunoglobulin variant having a deletion in a C-terminal multimerization-promoting region of at least one heavy chain, and may contain two or more immunoglobulin variants having different types of deletions.

In addition, the type of immunoglobulin constituting the immunoglobulin multimer of the present disclosure is not particularly limited, and may be selected from, for example, IgG, IgA, IgM, IgD, or IgE. In a preferred aspect, the immunoglobulin is IgA or IgM. In a preferred aspect, the immunoglobulin multimer of the present disclosure has a physiological activity, for example, a binding activity to an antigen.

The immunoglobulin multimer of the present disclosure may contain immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. In a preferred aspect, the immunoglobulin is a human immunoglobulin.

The immunoglobulin multimer of the present disclosure may contain, together with an immunoglobulin having a deletion in a C-terminal multimerization-promoting region, an immunoglobulin that does not have the deletion. The immunoglobulin having a deletion in a C-terminal multimerization-promoting region and the immunoglobulin that does not have the deletion may have a wild-type amino acid sequence in the heavy chain or light chain, or may have a mutation relative to the wild type. The mutation includes addition, deletion, and substitution of an amino acid, and includes shortening of an N-terminus or C-terminus. The properties such as antigen binding ability and stability of the immunoglobulin to be produced can be adjusted by the mutation.

In one aspect, the immunoglobulin constituting the immunoglobulin multimer of the present disclosure may be a mixture of structures of IgG, IgA, IgM, IgD, or IgE. For example, the immunoglobulin may be IgG, IgA, IgM, IgD, or IgE to which a multimerization-promoting region of IgA or IgM or another region is added by recombinant techniques, or in which a native region is partially replaced by such a region. Alternatively, the immunoglobulin constituting the immunoglobulin multimer of the present disclosure may be IgA or IgM in which a portion other than the multimerization-promoting region is replaced with that of another class of globulin. In the present disclosure, when it is stated that the immunoglobulin is IgG, IgA, IgM, IgD, or IgE, this includes each of IgG, IgA, IgM, IgD, and IgE that contains a part of another type of immunoglobulin and/or is partially replaced with a part of another type of immunoglobulin.

The immunoglobulin multimer of the present disclosure contains two or more immunoglobulins. In a preferred aspect, the immunoglobulin multimer of the present disclosure contains 2 to 3, 2 to 4, 2 to 5, 2 to 6, 2 to 7, 2 to 8, or 2 to 9 immunoglobulins.

In one aspect, the immunoglobulin variant contained in the immunoglobulin multimer of the present disclosure is an immunoglobulin variant described in the following (Immunoglobulin Variant Having Deletion in C-Terminal Multimerization-Promoting Region).

### (Immunoglobulin Variant Having Deletion in C-Terminal Multimerization-Promoting Region)

In one aspect, the present disclosure provides an immunoglobulin variant having a deletion in a C-terminal multimerization-promoting region. Typically, the immunoglobulin having a deletion in a C-terminal multimerization-promoting region of the present disclosure has the following structure.

An immunoglobulin variant having a deletion in a C-terminal multimerization-promoting region and having an ability to form an immunoglobulin multimer.

In one aspect, the immunoglobulin variant having a deletion in the C-terminal multimerization-promoting region of the present disclosure is used in the (Method for Producing Immunoglobulin Multimer) above.

Accordingly, the immunoglobulin variant having a deletion in the C-terminal multimerization-promoting region of the present disclosure may be an immunoglobulin variant having a deletion in a C-terminal multimerization-promoting region of at least one heavy chain, and may include two or more immunoglobulin variants having different types of deletions.

In addition, the type of immunoglobulin of the immunoglobulin variant having a deletion in the C-terminal multimerization-promoting region of the present disclosure is not particularly limited, and may be selected from, for example, IgG, IgA, IgM, IgD, or IgE. In a preferred aspect, the immunoglobulin variant is IgA or IgM. In a preferred aspect, the immunoglobulin variant of the present disclosure has a physiological activity, for example, a binding activity to an antigen.

The immunoglobulin variant of the present disclosure may contain immunoglobulins of various organisms. For example, the immunoglobulin may be an immunoglobulin of a human, a non-human primate, a mouse, a rat, a guinea pig, a rabbit, a hamster, a dog, a cat, a weasel, a cow, a pig, a horse, a deer, a wild boar, a sheep, a goat, a camel, or the like. In a preferred aspect, the immunoglobulin variant is a human immunoglobulin.

In one aspect, the immunoglobulin variant having a deletion in the C-terminal multimerization-promoting region of the present disclosure may have a mixed structure of IgG, IgA, IgM, IgD, or IgE. For example, the immunoglobulin may be IgG, IgA, IgM, IgD, or IgE to which a multimerization-promoting region of IgA or IgM or another region is added by recombinant techniques, or in which a native region is partially replaced by such a region. Alternatively, the immunoglobulin variant having a deletion in the C-terminal multimerization-promoting region of the present disclosure may be IgA or IgM in which a region other than the multimerization-promoting region is replaced with that of another class of globulin. In the present disclosure, when it is stated that the immunoglobulin variant is IgG, IgA, IgM, IgD, or IgE, this includes each of IgG, IgA, IgM, IgD, and IgE that contains a part of another type of immunoglobulin and/or is partially replaced with a part of another type of immunoglobulin.

The C-terminal multimerization-promoting region of the heavy chain of the immunoglobulin variant used in the method of the present disclosure includes a region having a function of promoting the multimerization process when the immunoglobulin forms a multimer of a dimer or more, or a region that stabilizes the formed multimer. The region may be a region included in a natural immunoglobulin, may also include an amino acid mutation or a heterologous molecule. Preferably, the region is a region included in a natural immunoglobulin.

The deletion in the C-terminal multimerization-promoting region of the heavy chain of the immunoglobulin variant includes any form that reduces or eliminates the multimerization-promoting function. In one aspect, the deletion removes a beta sheet structure in the C-terminal region of the immunoglobulin heavy chain that contributes to the multimerization-promoting function (Int J Mol Sci. 2021 Dec; 22(23): 12776). In one aspect, the deletion may be a deletion, substitution, or insertion of one or more amino acid sequences. In one aspect, the deletion may also be a binding of a heterologous molecule to the region. The position at which the deletion is present may be any position within the multimerization-promoting region. In a preferred aspect, the deletion is a deletion of an amino acid sequence, and a more preferred aspect is a deletion of one or more amino acids from the C-terminus. For example, when the immunoglobulin is mouse IgA, the deletion includes the amino acid sequence PTNVSVSVIMSEGDGICY (SEQ ID NO: 7) located at the C-terminus of the heavy chain.

The immunoglobulin variant used in the method of the present disclosure may include a heterologous amino acid sequence for promoting multimerization. As such a heterologous amino acid sequence, for example, an amino acid sequence forming a coiled-coil domain can be used. In one aspect, the heterologous amino acid sequence can be derived from any of cartilage oligomeric matrix protein (COMP), mannose-binding protein A, coiled-coil serine-rich protein 1, polypeptide release factor 2, SNAP-25, SNARE, Lac repressor, or apolipoprotein E. In a preferred aspect, as the heterologous amino acid sequence for promoting multimerization, for example, a coiled-coil assembly domain of cartilage oligomeric matrix protein (COMP) described in JP 2016-512309 A can be used. The heterologous amino acid sequence can be fused to an N-terminus or a C-terminus, preferably a C-terminus, of an immunoglobulin protein. Various linker sequences can be used for the fusion as long as a desired physiological function of the multimer immunoglobulin is not lost, and may not be used. In one aspect, a linker consisting of an amino acid sequence SSADDAKKDAAKKDDAKKDDAKKDAS (SEQ ID NO: 4) or a linker consisting of an amino acid sequence SSADDAAADAAAADDAAADDAAADAS (SEQ ID NO: 5) can be used. In one aspect, the heterologous sequence linker or linker for promoting multimerization can be fused to a shortened N-terminus or C-terminus of the immunoglobulin.

The class, amino acid sequence, and the like of the immunoglobulin having a deletion in the C-terminal multimerization-promoting region of the present disclosure are not particularly limited as described above, but as an example, the W27 antibody described in WO 2014/142084 A1 and a CHO cell-produced recombinant IgA of the W27 antibody may be used, and RS_H000_L001, in which a mutation for protein L binding is introduced into the light chain, may be used (see WO 2023/277142 A1, hereinafter, RS_H000_L001 is referred to as "W27 IgA"). More specifically, as an immunoglobulin derived from the W27 IgA antibody, an immunoglobulin having an amino acid deletion in the C-terminal region of the heavy chain and having a COMP sequence added thereto (CHO W27notailCOMP IgA) can be used.

The heavy chain amino acid sequence of CHO W27notailCOMP IgA expressed in mouse cells is shown below.

>SEQ ID NO: 1: CHO W27notailCOMP IgA H amino acid

sequence (without signal sequence)

EVQLQQSGSELVKSGASVKLSCTVSGFNFTDYYIHWVRQRTEQGLEWIGRIDPENDETT

YAPKFQGKATMTADTSSNTAYLQLTSLTSEDTAVYYCARSTVLDYWGHGTTLTVSSESA

RNPTIYPLTLPRALSSDPVIIGCLIHDYFPSGTMNVTWGKSGKDITTVNFPPALASGGG

YTMSSQLTLPAVECPEGESVKCSVQHDSNAVQELDVKCSGPPPPCPPCPPSCHPSLSLQ

RPALEDLLLGSDASLTCTLNGLRNPEGAVFTWEPSTGKDAVQKKAVQNSCGCYSVSSVL

PGCAERWNSGASFKCTVTHPESDTLTGTIAKITVNTFPPQVHLLPPPSEELALNELVSL

TCLVRAFNPKEVLVRWLHGNEELSPESYLVFEPLKEPGEGATTYLVTSVLRVSAELWKQ

GDQYSCMVGHEALPMNFTQKTIDRLSGKNSEGQIPLGGDLAPQMLRELQETNAALQDVR

ELLRQQVKEITFLKNTVMECDACGMQPARTPG*

Note that, when the immunoglobulin is expressed in mouse cells (for example, CHO), a signal sequence including a mouse IgV signal sequence at the N-terminus, such as the amino acid sequence MKCSWIIFFLMAVVTGVNS (SEQ ID NO: 6), can be added.

In addition, the light chain of CHO W27notailCOMP IgA has the same amino acid sequence as the light chain of the CHO cell-produced recombinant IgA variant (RS_H000_L001) of the W27 antibody, and the amino acid sequence of its light chain variable region (RS_LV001) includes a mutation for protein L binding. The amino acid sequence of the light chain variable region RS_LV001 is shown below.

>SEQ ID NO: 3: RS_LV001 light chain variable region

amino acid sequence

MKLPVRLLVLMFWIPGFSSDVLMTQSPASLSVSLGDRATISCRASQSIVHTNGNTYLEW
YLQKPGQSPKLLIYKVSNRFSGVPDRFSGSGSGTDFILKISRVEAEDLGVYYCFQGSHV
PPTFGGGTKLEVK*

### (Nucleic Acid Encoding Immunoglobulin Variant)

In one aspect, the present disclosure provides a nucleic acid encoding the immunoglobulin variant described in the (Immunoglobulin Variant Having Deletion in C-Terminal Multimerization-Promoting Region) above.

The nucleic acid may be a ribonucleotide or a deoxyribonucleotide. In addition, the nucleic acid may be in the form of a single strand or a double strand, and is not particularly limited.

The base sequence encoding the above-described amino acid sequence is not limited to a single base sequence for one amino acid sequence, and may be any base sequence determined by appropriately selecting codons encoding the amino acids according to the intended use of the nucleic acid. Examples of intended use include expressing the immunoglobulin having a deletion in the C-terminal multimerization-promoting region according to the present disclosure using the base sequence, and producing the immunoglobulin. In particular, various codons may be selected in consideration of the codon frequency corresponding to the type of host cell employed during production.

Such a base sequence can be easily determined in silico, for example, by using a publicly known program. In addition, the nucleic acid according to the present disclosure may also have a base sequence encoding the mutated amino acid sequence described in the (Immunoglobulin Having Deletion in C-Terminal Multimerization-Promoting Region) above, with respect to SEQ ID NOs: 1 to 50.

The nucleic acid encoding the immunoglobulin of the present disclosure can be produced, for example, by preparing base sequences encoding a heavy chain variable region and a light chain variable region, and base sequences encoding a heterologous heavy chain constant region and a light chain constant region, and by joining the base sequence encoding the prepared heavy chain variable region with the base sequence encoding the heavy chain constant region, and joining the base sequence encoding the prepared light chain variable region with the base sequence encoding the light chain constant region.

In one aspect, the immunoglobulin of the present disclosure is an antibody in which CDR1 to CDR3 of the heavy chain and/or light chain have amino acid sequences derived from a mouse, and the other regions have amino acid sequences derived from a human (this may also be referred to as a humanized antibody). The humanized antibody can be produced by preparing nucleic acids having base sequences encoding CDR1 to CDR3 of the heavy chain and/or light chain, and base sequences encoding the other regions, and recombining the nucleic acids so as to construct the heavy chain and the light chain. In one aspect, some bases may be substituted with other bases to maintain the binding ability of the antibody.

The base sequence contained in the nucleic acid of the present disclosure is not particularly limited, and for example, the base sequence: SEQ ID NO: 2 corresponding to the amino acid sequence described in the (Immunoglobulin Variant Having Deletion in C-Terminal Multimerization-Promoting Region) above: SEQ ID NO: 1 can be exemplified.

>SEQ ID NO: 2: Base sequence of base sequence of CHO
W27HnotailCOMP (including a region encoding the N-terminal
mouse IgV signal sequence “MKCSWIIFFLMAVVTGVNS”)

ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAAAATTCAGAGGGCCAGATCCCGCTGGGT
GGAGACCTGGCCCCACAGATGCTGCGAGAACTTCAGGAGACTAATGCGGCGCTGCAAGACGTGAGAG
AGCTGTTGCGACAGCAGGTCAAGGAGATCACCTTCCTGAAGAATACGGTGATGGAATGTGATGCTTG
CGGAATGCAGCCCGCACGCACTCCAGGCTGA

### (Vector)

In one aspect, the present disclosure provides a vector containing a nucleic acid encoding an immunoglobulin variant. The vector can be produced by incorporating a nucleic acid encoding an immunoglobulin variant into a vector known in the technical field. The production method is not particularly limited, and various methods known to those skilled in the art can be used.

The vector of the present disclosure can be used for expressing an immunoglobulin variant by being introduced into a host cell, and can also be used for genetic engineering applications such as in vitro use.

In one aspect, when the vector of the present disclosure is used as an expression vector, a phage vector, a viral vector, a plasmid vector, or the like can be used.

### (Cell)

In one aspect, the present disclosure provides a cell containing a nucleic acid encoding an immunoglobulin variant. The cell is not particularly limited, and includes cultured cells known in the technical field and cells obtained from a living body.

In one aspect, the cell is a cell used in the (Method for Producing Immunoglobulin Multimer) above, and expresses an immunoglobulin variant encoded by the nucleic acid. As the cell, various cells described in the (Method for Producing Immunoglobulin Multimer) above can be used.

In one aspect, the cell does not necessarily express the immunoglobulin variant encoded by the nucleic acid. For example, the cell may be a cell containing a nucleic acid encoding an immunoglobulin variant for the purpose of performing genetic engineering operations within the cell. A specific type of the cell is not particularly limited, and various cells known in the technical field can be used. In one aspect, the cell may be, for example, a mammalian cell, an avian cell, a reptilian cell, an amphibian cell, a fish cell, a fungal cell, a bacterial cell, or an archaeal cell, but is not limited thereto.

### (Composition)

In one aspect, the present disclosure provides a composition containing an immunoglobulin multimer or an immunoglobulin variant having a deletion in a C-terminal multimerization-promoting region.

The composition of the present disclosure can be provided as a composition containing a purified globulin multimer or immunoglobulin variant, a composition containing a host cell itself that expresses the immunoglobulin multimer or the immunoglobulin variant, a composition containing a part or all of a culture solution of a host cell that expresses the immunoglobulin multimer or the immunoglobulin variant, or a composition containing the host cell and a part or all of the culture solution. The composition of the present disclosure is preferably provided in the form of, but not limited to, a pharmaceutical composition, a food composition, a feed composition, a reagent composition, and the like. In one aspect, the pharmaceutical composition, the food composition, the feed composition, and the reagent composition as the composition of the present disclosure each include a material composition used to produce a final product.

### (Pharmaceutical Composition)

The pharmaceutical composition according to the present disclosure is used for the treatment of a disease, disorder, or condition by utilizing the effect of an immunoglobulin multimer or an immunoglobulin variant having a deletion in a C-terminal multimerization-promoting region.

In one aspect, the immunoglobulin multimer or the immunoglobulin variant according to the present disclosure binds to an in vivo pathogen associated with a disease, disorder, or condition, or to an in vivo molecule exhibiting an abnormal function, and exerts an effect of inhibiting the proliferation of the pathogen in vivo and/or promoting the excretion of the pathogen or the in vivo molecule exhibiting the abnormal function. A pharmaceutical composition containing such an immunoglobulin multimer or immunoglobulin variant is used for the prevention or treatment of a disease, disorder, or condition associated with the pathogen or the in vivo molecule exhibiting an abnormal function.

In the present disclosure, "treatment" of a disease, disorder or condition encompasses any treatment that ameliorates or suppresses the progression of the disease, disorder, or condition. In addition, in the present disclosure, "prevention" of a disease, disorder, or condition encompasses any prevention that prevents, delays, or alleviates the disease, disorder, or condition. In one aspect, the "prevention" encompasses an aspect of treating a subject predisposed to the "disease, disorder, or condition".

The pathogen encompasses any pathogen associated in vivo with a disease, disorder, or condition, and includes, for example, viruses, bacteria, fungi, and parasites, but is not limited thereto. The in vivo molecule expressing an abnormal function encompasses any in vivo molecule that exhibits an abnormal function associated with a disease, disorder, or condition. The in vivo molecule includes not only molecules originally present in a subject organism that exhibit an abnormal function due to changes in expression levels or structural mutations, but also molecules that are introduced into a body by pathogens or the like and exist therein.

In one aspect, the immunoglobulin multimer or the immunoglobulin variant having a deletion in the C-terminal multimerization-promoting region of the present disclosure includes W27 IgA and variants thereof, and exerts an effect of preventing or treating a disease, disorder, or condition by binding to C. difficile bacteria.

The disease associated with C. difficile is not particularly limited, examples thereof include an inflammatory bowel disease, ulcerative colitis, Crohn's disease, allergy, asthma, obesity, autoimmune disease, and neonatal necrotizing enterocolitis, and among them, an inflammatory bowel disease is preferable.

Such a pharmaceutical composition according to the present disclosure only needs to contain an effective amount of the immunoglobulin multimer or the immunoglobulin variant having a deletion in the C-terminal multimerization-promoting region according to the present disclosure, and for example, a content ratio of the antibody according to the present disclosure in 100 wt% of the pharmaceutical composition can be appropriately set within a range of 0.001 to 99.99 wt%, in consideration of the type of a target disease, a dosage form, an administration method, an administration target, a degree of symptoms of an administration subject, a degree of effect exerted by administration, and the like.

As used in the present specification, the term "effective amount" refers to an amount of the immunoglobulin multimer or the immunoglobulin variant having a deletion in the C-terminal multimerization-promoting region according to the present disclosure that exerts a desired physiological effect in a living body.

The pharmaceutical composition according to the present disclosure may further contain a pharmaceutically acceptable carrier or additive in combination with the immunoglobulin multimer or the immunoglobulin variant having a deletion in the C-terminal multimerization-promoting region of the present disclosure. The pharmaceutically acceptable carrier or additive means any carrier, diluent, excipient, suspending agent, lubricant, adjuvant, medium, delivery system, emulsifier, disintegrant, absorbent, preservative, surfactant, colorant, flavoring, or sweetener, and a known pharmaceutically acceptable carrier or additive may be employed.

Examples of such an administration target include, but are not limited to, mammals such as a human, a mouse, a rat, a guinea pig, a rabbit, a hamster, a dog, a cat, a weasel, a cow, and a pig, and birds such as a chicken.

A dosage and administration method of the pharmaceutical composition vary depending on the type of disease, gender, species, age, general condition, severity of the disease, degree of desired effect, and the like that affects the administration target. Usually, the dosage may be appropriately set in a range of 0.001 to 100 mg/kg/day.

In addition, the administration method is not particularly limited, but it is preferable to directly administer to mucosal tissue, and examples of such an administration method include oral administration, intranasal administration, and enteral administration.

Note that the enteral administration is not limited to administration via the anus, and includes, for example, administration via a tube or the like inserted into the digestive tract from the outside of the individual like a gastrostomy or the like. A position where the digestive tract is inserted is not limited to the intestine, and examples thereof include esophagus, stomach, small intestine (including duodenum, jejunum, ileum, and the like), and large intestine (including cecum, colon, rectum, and the like).

Note that in the administration of such a pharmaceutical composition according to the present disclosure, the above amount may be administered once a day, or may be administered in several divided doses. In addition, an administration interval may be every day, every other day, every week, every other week, every 2 or 3 weeks, every month, every other month, or every 2 or 3 months as long as the therapeutic effect on the disease is obtained.

### (Food Composition and Feed Composition)

The food composition or feed composition according to the present disclosure contains an immunoglobulin multimer or an immunoglobulin variant having a deletion in a C-terminal multimerization-promoting region according to the present disclosure. The food composition according to the present disclosure includes, in addition to a general food, all forms of foods ingested as foods, such as a food for specified health uses including a food for conditional specified health uses, a nutritional supplementary food, a functional food, and a food for a patient. By using such a food composition or feed composition, it is possible to utilize the physiological activity of the immunoglobulin multimer or the immunoglobulin variant having a deletion in the C-terminal multimerization-promoting region according to the present disclosure. The food composition can be provided as a food composition labeled as for intestinal regulation, intestinal environment improvement, intestinal environment optimization, intestinal spoilage prevention, or the like.

A blending ratio of the immunoglobulin multimer or the immunoglobulin variant having a deletion in the C-terminal multimerization-promoting region in such a food composition or feed composition is not particularly limited and may be appropriately adjusted according to the form, use, and the like of the food composition or feed composition. Usually, the blending ratio may be about 0.001 to 99 wt% with respect to the total amount of the composition.

The intake amount of the food composition or the feed composition according to the present disclosure is not particularly limited, and can be set according to an intended effect, an intended degree thereof, other various conditions, and the like. For example, in terms of the amount of the immunoglobulin multimer according to the present disclosure, it is generally sufficient to administer about 0.001 to 100 mg/kg/day, and the amount may be taken once or several times per day.

Specific examples of the form of the food composition described above include, but are not limited to, drinks such as soft drinks, carbonated drinks, nutritional drinks, fruit drinks, lactic acid drinks, and milk drinks; frozen desserts such as ice cream, ice sorbet, and shaved ice; confectioneries such as candies, candies, gum, chocolate, tablet candies, snacks, biscuits, jelly, jam, cream, and baked confectioneries; noodles such as buckwheat noodles, thick noodles made from wheat flour, starch noodles, Chinese-style noodles, and instant noodles; fish or livestock processed foods such as fish cakes, ham, and sausage; dairy products such as processed milk products and fermented milk products; oil and fat and oil and fat processed foods such as salad oil, tempura oil, margarine, mayonnaise, shortening, whipped cream, and dressing; seasonings such as sauces and tare sauce; and soups, stews, salads, side dishes, furikake, pickles, bread, and cereal. In addition, in the cases of foods for specified health uses, nutritional supplementary foods, functional foods, and the like, examples thereof include powders, granules, capsules, troches, tablets, and syrups.

The food composition or the feed composition according to the present disclosure may be provided as a composition containing an immunoglobulin multimer or an immunoglobulin variant having a deletion in a C-terminal multimerization-promoting region, a composition containing a host cell itself that expresses an immunoglobulin or multimer immunoglobulin, a composition containing a part or all of a culture solution of a host cell that expresses the immunoglobulin multimer or the immunoglobulin variant having a deletion in the C-terminal multimerization-promoting region, or a composition containing the host cell and a part or all of the culture solution. In one aspect, the food composition or the feed composition of the present disclosure is provided as a composition produced by a culture process of a host cell, such as a fermentation process. In this case, a culture or a brewed and fermented product may be used as a food composition as it is, or a product obtained by purifying a part of the brewed and fermented product may be used as a food composition. For example, it is possible to filter a solid component containing a host cell from a culture or a brewed and fermented product and provide the resulting liquid as a liquid composition. Such a composition may contain the host cell itself, a part of the host cell, an extract of the host cell, a component serving as a culture medium for the host cell, or a mixture thereof. Non-limiting examples of such a food composition in which a filamentous fungus is used as the host cell include mirin, sake, amazake, miso, soy sauce, salt koji, rice koji, soy sauce koji, barley koji, soybean koji, koji natto, sake lees fish koji: fish sauce base, ragi (Indonesia), ketchup (Indonesian miso with no tomatoes), soy sauce: hishio and jiu niang, sweet fermented rice, mochi koji, maki koji, cheonggukjang, or katsuobushi, and other miscellaneous.

The feed composition of the present disclosure can be provided as a feed composition labeled as for intestinal regulation, intestinal environment improvement, intestinal environment optimization, intestinal spoilage prevention, or the like for various animals.

The specific form of the feed composition described above is not particularly limited, and for example, as long as the effect exhibited by the feed composition according to the present disclosure described above is not impaired, a feed composition may be prepared by mixing it with normal feed, or, as necessary, by mixing it with components that can be blended with normal feed, and the feed composition itself may also be used as feed. For example, a feed may be prepared by blending the lees obtained from the production of a fermented food using the host cell.

### (Reagent Composition)

The reagent composition of the present disclosure is provided as a composition that utilizes the physiological activity of an immunoglobulin multimer or an immunoglobulin variant having a deletion in a C-terminal multimerization-promoting region. For example, it is provided as a reagent that utilizes the specific antigen binding activity of the immunoglobulin multimer or the immunoglobulin variant having a deletion in the C-terminal multimerization-promoting region, and the activity of agglutinating the bound target. Such a reagent composition can be used for purposes such as detection, quantification, collection, or removal of a target molecule.

### (Method for Preventing or Treating Disease or the Like)

In one aspect, the present disclosure provides a method for preventing or treating a disease, disorder, or condition. Typically, the method for preventing or treating a disease, disorder, or condition of the present disclosure utilizes the physiological activity of the immunoglobulin multimer or the immunoglobulin variant having a deletion in the C-terminal multimerization-promoting region according to the present disclosure. The immunoglobulin multimer or the immunoglobulin variant having a deletion in the C-terminal multimerization-promoting region according to the present disclosure binds to an in vivo pathogen associated with a disease, disorder, or condition, or to an in vivo molecule exhibiting an abnormal function, and exerts an effect of inhibiting the proliferation of the pathogen in vivo and/or promoting the excretion of the pathogen or the in vivo molecule exhibiting the abnormal function. By utilizing the physiological activity of such an immunoglobulin multimer or an immunoglobulin having a deletion in a C-terminal multimerization-promoting region, a method for preventing or treating a disease, disorder, or condition associated with the pathogen or in vivo molecule exhibiting an abnormal function is provided to a subject in need of such prevention or treatment.

In one aspect, the method for preventing or treating a disease, disorder, or condition of the present disclosure includes the following configuration.

A method for preventing or treating a disease, disorder, or condition associated with a pathogen or an in vivo molecule exhibiting an abnormal function, the method including administering,
to a subject in need of prevention or treatment of the disease, disorder, or condition associated with the pathogen or the in vivo molecule exhibiting an abnormal function,
a composition containing an immunoglobulin multimer or an immunoglobulin having a deletion in a C-terminal multimerization-promoting region, that binds to the pathogen or the in vivo molecule exhibiting an abnormal function.

In one aspect, the method for preventing or treating a disease, disorder, or condition of the present disclosure includes W27 IgA and a variant thereof as an immunoglobulin multimer or an immunoglobulin variant, and provides a method for preventing or treating a disease, disorder, or condition associated with C. difficile bacteria by utilizing the action of the immunoglobulin multimer or immunoglobulin variant binding to C. difficile bacteria.

The method for preventing or treating a disease, disorder, or condition of the present disclosure includes a step of administering, to a subject in need of prevention or treatment of the disease, disorder, or condition, an immunoglobulin multimer or an immunoglobulin variant having a deletion in a C-terminal multimerization-promoting region of the present disclosure.

The administration mode, administration target, and disease described above may be the same as those described in the (Pharmaceutical Composition) above.

### (Test, Examination, and Diagnostic Method)

In one aspect, the present disclosure provides a test, examination, and diagnostic method. Typically, the test, examination, and diagnostic method of the present disclosure utilizes the specific binding activity of the immunoglobulin multimer or the immunoglobulin variant having a deletion in the C-terminal multimerization-promoting region according to the present disclosure. The immunoglobulin multimer or the immunoglobulin variant having a deletion in the C-terminal multimerization-promoting region according to the present disclosure can specifically bind to a target molecule in a test sample, an examination sample, or in vivo tissue. A test or examination method is provided by utilizing a specific binding activity of an immunoglobulin multimer or an immunoglobulin variant having a deletion in a C-terminal multimerization-promoting region, for determining the presence or absence and/or quantifying the amount of target molecule in a test sample, an examination sample, or in vivo tissue. Furthermore, in one aspect, a test method or diagnostic method is provided for determining a condition, disease, prognosis, or disease risk of a subject based on the results of the above test or examination.

In one aspect, the test, examination, and diagnostic method of the present disclosure includes the following configuration.

A test, examination, and diagnostic method comprising:
a step of bringing a test sample, an examination sample, or in vivo tissue into contact with an immunoglobulin multimer or an immunoglobulin variant having a deletion in a C-terminal multimerization-promoting region; and
a step of detecting or quantifying a molecule that binds to the immunoglobulin multimer or the immunoglobulin variant having a deletion in the C-terminal multimerization-promoting region in the test sample, examination sample, or in vivo tissue.

### Examples

Hereinafter, the present disclosure will be described in more detail with reference to Examples, but these Examples are merely illustrative and do not limit the present disclosure.

### Methods and Materials

### 1. DNA Manipulation Method

E. coli DH5α was used for DNA manipulation. PrimeSTARHS DNA polymerase (TaKaRa) was used for PCR. InFusion HD cloning kit (TaKaRa) was used for plasmid production. Plasmids were extracted by alkaline SDS method. The base sequence was analyzed by FASMAC Co., Ltd.

### 2. Quantification of Immunoglobulin and Non-Reducing SDS-PAGE

The quantification of the purified immunoglobulin was performed by ELISA. The details of the immunoglobulin quantification method are as follows. First, Anti-goat-mouse IgA (Southern Biotech: 1040-01) was diluted with 0.05 M Na2CO3 to 2 µg/ml, added to each well of C96 MaxiSorp Nunc-Immuno Plate (Thermo Fisher) (hereinafter, referred to as ELISA plate) by 50 µl/well, and immobilized overnight at 4°C. The plate was washed 3 times with PBS using Vaccu-Pette/96 multi-well pipetter (Sigma-Aldrich) (hereinafter, plate washer), then PBS (PBS-BSA) containing 1% bovine serum albumin (BSA) (Wako) was added so as to be 150 µl/well, and the plate was allowed to stand at 4°C overnight to perform blocking. A series of antibody fluids was prepared in a 96-well plate. A dilution series of the antibody fluids was prepared using PBS-BSA at equal magnification (x1, x1/100 and x1/1,000 of the purified antibodies are defined as x1), 3-fold (x1/3), 10-fold (x1/10), 30-fold (x1/30), 100-fold (x1/100), 300-fold (x1/300), 1,000-fold (x1/1,000), and 3,000-fold (x1/3,000). After PBS-BSA was completely removed from the plate, the serial dilution was added so as to be 50 µl/well, and the plate was allowed to stand at room temperature for 1 hour for reaction.

Next, the plate was washed 3 times with a plate washer with PBS (PBS-T) containing 0.05%Tween 20 (Chem Cruz), and then, a secondary antibody Alkaline Phosphatase (ALP)-conjugated anti-goat mouse IgA (Southern Biotech: 1040-04) was diluted with PBS-BSA to a final concentration of 0.5 µg/ml, added at 50 µl/well, and allowed to stand at room temperature for 1 hour for reaction. The plate was washed 3 times with a plate washer using PBS-T, and then a substrate solution prepared by adding 10 µl of 1 M MgCl2 and 1 tablet of Substrate for Alkaline Phosphatase (Sigma-Aldrich) to 5 ml of a carbonate buffer (6.5 mM Na2CO3, 18.5 mM NaHCO3) was added at 50 µl/well. After the mixture was left to stand while being shielded from light at room temperature, it was visually confirmed that the color was sufficiently developed, and absorbance at an optical density (O.D.) of 405 nm was measured using TriStar2 LB942 (BERTHOLD TECHNOLOGIES).

Separately from the above, it was confirmed whether an immunoglobulin multimer was produced by non-reducing SDS-PAGE. For sample preparation, 3 × Native buffer (0.2 M Tris-HCl pH 6.8, 30% Glycerol, 0.015% Bromophenol blue) was added to the immunoglobulin (5 µg). Using 6% SDS-PAGE gel using WIDE RANGE Gel Preparation Buffer (4×) for PAGE (Nacalai Tesque), electrophoresis was performed, and then, Coomassie brilliant blue (CBB) staining was performed. In the reduced SDS-PAGE, a 2-ME-added buffer was added to a sample, the sample was denatured at 95°C for 10 minutes, the sample was electrophoresed using 6% SDS-PAGE gel as in the case of non-reduction, and then, Coomassie brilliant blue (CBB) staining was performed.

### 3. Analysis of Binding Ability of Immunoglobulin Multimers to E. coli

In order to compare the binding ability of the immunoglobulin multimers, ELISA against E. coli was performed. E. coli (DH5α or BW38092 and a variant thereof, all of which are K12 strains) was subjected to aerobic culture (LB medium) at 37°C overnight and collected by centrifugation. After washing the bacterial cell body with PBS, bacteria were suspended in a 0.05 M Na2CO3 buffer and coated onto an ELISA plate (4°C overnight). As in the purified IgA antibody quantitative ELISA, after blocking with PBS containing 1% BSA, each antibody dilution series in a 96-well plate was prepared and added, and reacted at room temperature for about 1 hour. Thereafter, the plate was washed with 0.05% Tween 20-added PBS, and a secondary antibody Alkaline Phosphatase (ALP)-conjugated anti-goat mouse IgA (Southern Biotech: 1040-04) was diluted with PBS-BSA to a final concentration of 0.5 µg/ml, added at 50 µl/well, and allowed to stand at room temperature for 1 hour for reaction. After washing the plate three times with a plate washer with PBS-T, a color developing reaction was performed in the same manner as described above using Alkali Phosphatase tablet (Sigma). For sufficient reaction, the ELISA plate after color development was reacted at 4 degrees overnight, and OD405 nm was measured using TriStar2 LB942 (BERTHOLD TECHNOLOGIES).

### 4. In Vitro Growth Inhibition Assay of Immunoglobulin Multimers Against Clostridioides difficile (C. difficile) Bacteria

The C. difficile anaerobically cultured overnight was diluted to 10,000 cfu per tube, each test antibody was added to a final concentration of 2.4 mg/ml, and anaerobic culture was performed. After 6 hours, the bacterial suspension was diluted, and after plating, the number of colonies was measured by further anaerobic culture. As test antibodies, a pentameric IgA antibody of rW27 (a pentameric IgA antibody composed of an IgA having a heavy chain with SEQ ID NO: 4: CHO W27notailCOMP and a light chain of antibody RS_H000_L001 (see WO 2014/142084 A1)), a dimeric IgA antibody of rW27 (a dimeric IgA antibody composed of an IgA having a heavy chain and a light chain of antibody RS_H000_L001 (see WO 2014/142084 A1) and a J chain), and a control dimeric IgA antibody were used.

### 5. Suppressive Effect of Immunoglobulin Multimers on Enteritis Caused by C. difficile Bacteria

The enteritis suppressive effect was tested by orally administering the test antibody to mice by a method similar to that described in WO 2023/277166 A1.

Specifically, a spore suspension of C. difficile strain (VPI10483) was prepared, aliquoted, and stored at - 80°C. The number of viable bacteria obtained by thawing the spore liquid, culturing the thawed spore liquid, and then seeding the thawed spore liquid on C. difficile selective medium plate (TCCFA plate) was determined in advance.

The preparation method of spores is as follows.

C. difficile was plated on SMC medium and anaerobically cultured at 37°C for 7 days. 3 ml of ice-cold sterile water was added to the plate, and colonies were scraped off with a cell scraper and transferred to a 50 ml tube. Further, the plate was washed with 3 ml of ice-cold sterile water, and the washing liquid was also transferred to the same tube. After centrifugation at 8,000 g and 4°C for 10 minutes, the supernatant was discarded, and the bacterial bodies were suspended in 20 ml of ice-cold sterile water. After centrifugation again at 8,000 g and 4°C for 10 minutes, the supernatant was discarded, and the bacterial bodies were suspended in 10 ml of ice-cold sterile water. After the suspension, 10 ml of ethanol was added, stirring was performed by vortex, and the obtained product was allowed to stand at room temperature for 1 hour. Centrifugation was performed at 8,000 g and 4°C for 10 minutes, the supernatant was discarded, and the bacterial bodies were suspended in 20 ml of ice-cold sterile water. This operation was repeated twice, and the supernatant was discarded and then suspended in 5 ml of ice-cold sterile water. Each 100 µl was dispensed into a 1.5 ml tube and stored at -80°C. The SMC medium composition is shown below.

### SMC Medium Composition

| Reagent name | Amount used |
|---|---|
| Bacto Peptone | 18 g |
| Bacto Proteose Peptone | 1 g |
| Ammonium sulfate | 200 mg |
| Tris | 300 mg |
| Agar | 3 g |

The reagents were dissolved in 200 ml of sterile distilled water and sterilized in an autoclave. When the medium was cooled to about 60°C, 600 µl of 10% (w/v) L-cysteine was added, and the medium was dispensed in an appropriate amount onto a 10 cm plate.

In the C. difficile selective medium plate, the reagents in Table 1 were dissolved in 400 ml of sterile distilled water and sterilized in an autoclave. The medium was naturally cooled to 60°C, 2 ml of Cycloserine (Sigma-Aldrich) (50 mg/ml) and 2 ml of Cefoxitin (Sigma-Aldrich) (1.6 mg/ml) were added thereto, and the medium was dispensed in appropriate amounts onto 10 cm plates to prepare them.

**(Table 1) Composition of C. difficile selective medium**

| Reagent name | Amount used |
|---|---|
| Bacto Proteose Peptone | 20 g |
| Disodium hydrogen phosphate dodecahydrate | 2.68 g |
| Potassium dihydrogen phosphate | 0.4 g |
| Sodium chloride | 0.8 g |
| Magnesium sulfate | 0.048 g |
| D- (-) -Fructose | 2.4 g |
| Taurocholic acid sodium salt hydrate | 0.4 g |
| Agar | 6.64 g |
| Neutral Red Solution (0.33%) | 1 ml |

C57BL/6 mice (8-week-old) were purchased from CLEA Japan, Inc., and acclimatized in a sterile isolator for an infection experiment for 1 week, and then 3 types of mixed antibiotics (Gentamicin: 500 mg/kg, Kanamycin: 150 mg/kg, Metronidazole: 50 mg/kg) were orally administered for 4 days using a feeding tube. After 4 days, the body weight of each mouse was measured, individuals in which a weight loss occurred by antibiotic administration were excluded, and the remaining mice were randomly grouped. On the day after the completion of antibiotic administration, each mouse was orally infected with C. difficile spores (10 x 10³ cfu) using a feeding tube. After 6 hours, each test antibody was orally administered at 300 µg per mouse using a feeding tube (n = 6 mice per test antibody). As test antibodies, a pentameric IgA antibody of rW27 (a pentameric IgA antibody composed of an IgA having a heavy chain with SEQ ID NO: 4: CHO W27notailCOMP and a light chain of antibody RS_H000_L001 (see WO 2014/142084 A1)), a dimeric IgA antibody of rW27 (a dimeric IgA antibody composed of an IgA having a heavy chain and a light chain of antibody RS_H000_L001 (see WO 2014/142084 A1) and a J chain), and a control dimeric IgA antibody were used. Thereafter, 300 µg of the antibody was administered once a day for a total of 7 days. From day 8 onward, progress was observed without antibody administration.

### Example 1: Production of Immunoglobulin Having Deletion in C-Terminal Multimerization-Promoting Region

The effect on multimerization was examined for an immunoglobulin having a deletion in the C-terminal multimerization-promoting region.

The gene encoding the immunoglobulin and a vector containing the same were prepared in accordance with standard methods, and introduced into CHO cells in the same manner as described in Example 8 of WO 2023/277166 A1.

As an exemplary immunoglobulin, the W27 antibody described in WO 2014/142084 A1 and a CHO cell-produced recombinant IgA of the W27 antibody in which a mutation for protein L binding was introduced into the light chain, RS_H000_L001 (W27 IgA), were used (see Japanese Patent Application No. 2021-110421).

Using the W27 IgA antibody, genome-modified CHO cells were produced by standard methods using a nucleic acid construct encoding IgA containing a natural L chain and a fusion protein having the following configuration as an H chain, and the multimer formation state of secreted IgA was examined.

| Construct name | Structure of H chain | H chain amino acid sequence name/SEQ ID NO: |
|---|---|---|
| AAcomp | COMP sequence is fused to C-terminus of full-length H chain via linker consisting of amino acid sequence SSADDAAADAAAADDAAADDAAADAS. | SEQ ID NO: 8 |
| KKcomp | COMP sequence is fused to C-terminus of full-length H chain via linker consisting of amino acid sequence SSADDAKKDAAKKDDAKKDDAKKDAS. | SEQ ID NO: 9 |
| direct-comp | COMP sequence is fused to C-terminus of full-length H chain without linker. | SEQ ID NO: 10 |
| notail-comp | COMP sequence is fused to C-terminus of H chain lacking tail piece without linker. | SEQ ID NO: 1 |

The amino acid sequences of the IgA heavy chains having each structure and the nucleic acid sequences encoding them are shown below. Note that, for expression in CHO cells, a mouse IgV signal sequence "MKCSWIIFFLMAWTGVNS" was added to the N-terminus.
>SEQ ID NO: 8: CHO W27 HAACOMP IgA H chain amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYSSADDAAADAAAA
DDAAADDAAADASNSEGQIPLGGDLAPQMLRELQETNAALQDVRELLRQQVKEITFLKN
TVMECDACGMQPARTPG*
>SEQ ID NO: 9: CHO W27 HKKCOMP IgA H chain amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYSSADDAKKDAAAA
DDKKADDAKKDASNSEGQIPLGGDLAPQMLRELQETNAALQDVRELLRQQVKEITFLKN
TVMECDACGMQPARTPG*
>SEQ ID NO: 10: CHO W27HdirectCOMP IgA H amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKPTNVSVSVIMSEGDGICYNSEGQIPLGGDLA
PQMLRELQETNAALQDVRELLRQQVKEITFLKNTVMECDACGMQPARTPG*
>SEQ ID NO: 1 (supra): CHO W27notailCOMP IgA H amino acid sequence (without signal sequence) 
EVQLQQSGSELVKSGASVKLSCTVSGFNFTDYYIHWVRQRTEQGLEWIGRIDPENDETT
YAPKFQGKATMTADTSSNTAYLQLTSLTSEDTAVYYCARSTVLDYWGHGTTLTVSSESA
RNPTIYPLTLPRALSSDPVIIGCLIHDYFPSGTMNVTWGKSGKDITTVNFPPALASGGG
YTMSSQLTLPAVECPEGESVKCSVQHDSNAVQELDVKCSGPPPPCPPCPPSCHPSLSLQ
RPALEDLLLGSDASLTCTLNGLRNPEGAVFTWEPSTGKDAVQKKAVQNSCGCYSVSSVL
PGCAERWNSGASFKCTVTHPESDTLTGTIAKITVNTFPPQVHLLPPPSEELALNELVSL
TCLVRAFNPKEVLVRWLHGNEELSPESYLVFEPLKEPGEGATTYLVTSVLRVSAELWKQ
GDQYSCMVGHEALPMNFTQKTIDRLSGKNSEGQIPLGGDLAPQMLRELQETNAALQDVR
ELLRQQVKEITFLKNTVMECDACGMQPARTPG*
>SEQ ID NO: 11: Base sequence of CHO W27HAACOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACTCCTCCGCTGACGATGCCGCTGCTGATGCTGCCGCCGCCG
ATGATGCTGCTGCTGACGACGCCGCTGCTGACGCTTCCAACTCCGAGGGCCAGATCCCGCTGGGTGG
AGACCTGGCCCCACAGATGCTGCGAGAACTTCAGGAGACTAATGCGGCGCTGCAAGACGTGAGAGAG
CTGTTGCGACAGCAGGTCAAGGAGATCACCTTCCTGAAGAATACGGTGATGGAATGTGATGCTTGCG
GAATGCAGCCCGCACGCACTCCAGGCTGA
>SEQ ID NO: 12: Base sequence of base sequence of CHO W27HKKCOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACAGCTCGGCAGACGATGCCAAGAAAGATGCAGCCAAGAAAG
ACGATGCCAAGAAAGACGACGCCAAGAAAGACGCTTCCAATTCAGAGGGCCAGATCCCGCTGGGTGG
AGACCTGGCCCCACAGATGCTGCGAGAACTTCAGGAGACTAATGCGGCGCTGCAAGACGTGAGAGAG
CTGTTGCGACAGCAGGTCAAGGAGATCACCTTCCTGAAGAATACGGTGATGGAATGTGATGCTTGCG
GAATGCAGCCCGCACGCACTCCAGGCTGA
>SEQ ID NO: 13: Base sequence of base sequence of CHO W27HdirectCOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACAATTCAGAGGGCCAGATCCCGCTGGGTGGAGACCTGGCCC
CACAGATGCTGCGAGAACTTCAGGAGACTAATGCGGCGCTGCAAGACGTGAGAGAGCTGTTGCGACA
GCAGGTCAAGGAGATCACCTTCCTGAAGAATACGGTGATGGAATGTGATGCTTGCGGAATGCAGCCC
GCACGCACTCCAGGCTGA
>SEQ ID NO: 2: Base sequence of base sequence of CHO W27HnotailCOMP (including N-terminal signal sequence)

ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAAAATTCAGAGGGCCAGATCCCGCTGGGT
GGAGACCTGGCCCCACAGATGCTGCGAGAACTTCAGGAGACTAATGCGGCGCTGCAAGACGTGAGAG
AGCTGTTGCGACAGCAGGTCAAGGAGATCACCTTCCTGAAGAATACGGTGATGGAATGTGATGCTTG
CGGAATGCAGCCCGCACGCACTCCAGGCTGA

Note that the light chain of the W27 IgA antibody has the same amino acid sequence as the light chain of the CHO cell-produced recombinant IgA variant (RS_H000_L001) of the W27 antibody (see WO 2023/277142 A1), and the amino acid sequence of its light chain variable region (RS_LV001) includes a mutation for protein L binding.
>RS_LV001 (SEQ ID NO: 3) (supra)

MKLPVRLLVLMFWIPGFSSDVLMTQSPASLSVSLGDRATISCRASQSIVHTNGNTYLEW
YLQKPGQSPKLLIYKVSNRFSGVPDRFSGSGSGTDFILKISRVEAEDLGVYYCFQGSHV
PPTFGGGTKLEVK*

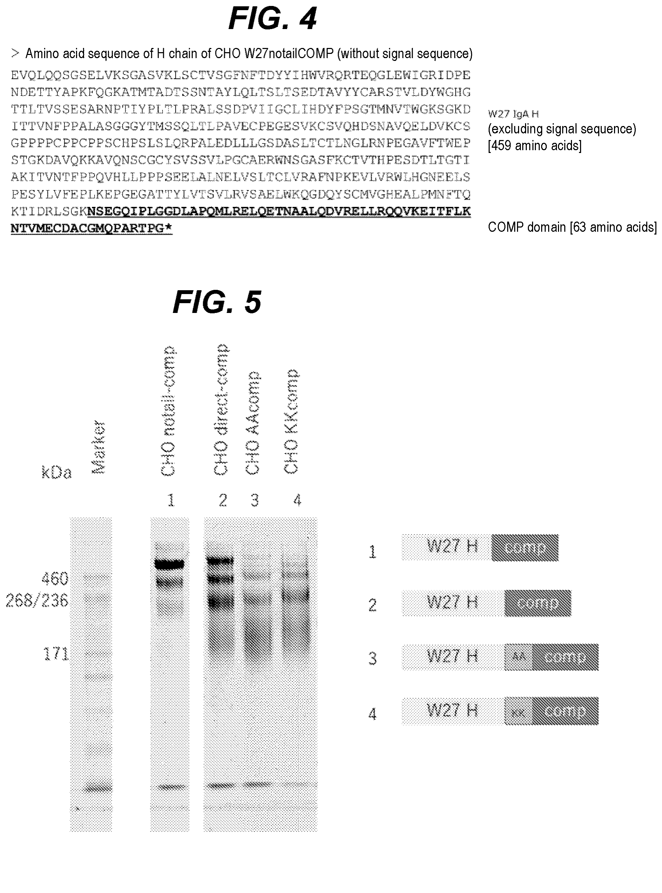

As shown in Fig. 5, it was confirmed that the structure of the H chain C-terminal region of IgA and the structure of the linker can affect the multimer formation of IgA. In particular, it was observed that, in a case where the H chain to which the COMP sequence was bound was used without using the linker, particularly in a case where the H chain to which the COMP sequence was bound by partially deleting the C-terminal region of IgA was used, a ratio of IgA produced as a multimer increased.

### Example 2: Binding Properties of Immunoglobulin Multimers

As the immunoglobulin multimer, the binding ability of multimerized W27 IgA to E. coli was confirmed. Note that, as described in WO 2014/142084 A1, it is known that the W27 IgA antibody expressed in mammalian cells binds to E. coli. The multimerized W27 IgA secreted from CHO cells was purified, and the ability of these IgA multimers to bind to E. coli was tested. Note that, for comparison, a W27 IgA monomer (W27 monomer) and a W27 IgA dimer (W27 dimer), obtained by expressing unmodified W27 in CHO cells, were simultaneously tested.

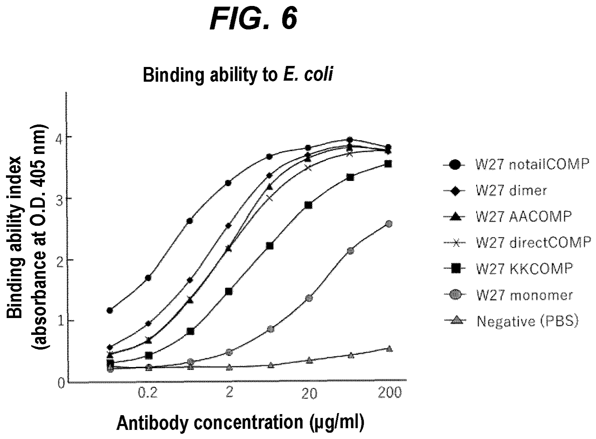

As a result of measuring the binding ability to E. coli, as shown in FIG. 6, all of the W27 IgA antibody multimers secreted from CHO cells exhibited significantly higher binding ability compared to the W27 IgA monomer (W27 monomer) and the W27 IgA dimer (W27 dimer). Furthermore, among the W27 IgA antibody multimers secreted from CHO cells, W27notailCOMP, which lacks the C-terminal region and is fused to COMP without a linker, exhibited the highest binding to E. coli.

### Example 3: In Vitro Growth Inhibition Effect of Immunoglobulin Multimers on C. difficile Bacteria

As the immunoglobulin multimer, the growth inhibition effect of multimerized W27 IgA against C. difficile bacteria was tested.

As a result, both the rW27 pentameric IgA antibody and the rW27 dimeric IgA antibody exhibited superior growth inhibition effects against C. difficile bacteria compared to the control dimeric IgA antibody.

These results indicate that the rW27 pentameric IgA antibody and the rW27 dimeric IgA antibody, which are immunoglobulin multimers, have excellent growth inhibition effects against C. difficile bacteria in vitro.

### Example 4: In Vivo Suppressive Effect of Immunoglobulin Multimers on C. difficile-Associated Enteritis

The suppressive effects of the rW27 pentameric IgA antibody and the rW27 dimeric IgA antibody, as immunoglobulin multimers, on C. difficile-associated enteritis were confirmed by in vivo administration testing.

As a result, the rW27 dimeric IgA antibody exhibited a slight improvement in survival rate compared to the control dimeric IgA antibody. On the other hand, the rW27 pentameric IgA antibody exhibited a remarkably excellent survival rate compared to the rW27 dimeric IgA antibody and the control dimeric IgA antibody (p = 0.0295).

As a result, it was revealed that, in particular, the rW27 pentameric IgA antibody has an excellent suppressive effect on C. difficile-associated enteritis in vivo.

The sequence information described in the present disclosure is shown again below.
>SEQ ID NO: 1: CHO W27notailCOMP IgA H amino acid sequence (without signal sequence) 
EVQLQQSGSELVKSGASVKLSCTVSGFNFTDYYIHWVRQRTEQGLEWIGRIDPENDETTYAPKFQGK
ATMTADTSSNTAYLQLTSLTSEDTAVYYCARSTVLDYWGHGTTLTVSSESARNPTIYPLTLPRALSS
DPVIIGCLIHDYFPSGTMNVTWGKSGKDITTVNFPPALASGGGYTMSSQLTLPAVECPEGESVKCSV
QHDSNAVQELDVKCSGPPPPCPPCPPSCHPSLSLQRPALEDLLLGSDASLTCTLNGLRNPEGAVFTW
EPSTGKDAVQKKAVQNSCGCYSVSSVLPGCAERWNSGASFKCTVTHPESDTLTGTIAKITVNTFPPQ
VHLLPPPSEELALNELVSLTCLVRAFNPKEVLVRWLHGNEELSPESYLVFEPLKEPGEGATTYLVTS
VLRVSAELWKQGDQYSCMVGHEALPMNFTQKTIDRLSGKNSEGQIPLGGDLAPQMLRELQETNAALQ
DVRELLRQQVKEITFLKNTVMECDACGMQPARTPG*
>SEQ ID NO: 2: Base sequence of base sequence of CHO W27HnotailCOMP (including a region encoding the N-terminal mouse IgV signal sequence "MKCSWIIFFLMAVVTGVNS") 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAAAATTCAGAGGGCCAGATCCCGCTGGGT
GGAGACCTGGCCCCACAGATGCTGCGAGAACTTCAGGAGACTAATGCGGCGCTGCAAGACGTGAGAG
AGCTGTTGCGACAGCAGGTCAAGGAGATCACCTTCCTGAAGAATACGGTGATGGAATGTGATGCTTG
CGGAATGCAGCCCGCACGCACTCCAGGCTGA
>SEQ ID NO: 3: RS_LV001 light chain variable region amino acid sequence 
MKLPVRLLVLMFWIPGFSSDVLMTQSPASLSVSLGDRATISCRASQSIVHTNGNTYLEW
YLQKPGQSPKLLIYKVSNRFSGVPDRFSGSGSGTDFILKISRVEAEDLGVYYCFQGSHV
PPTFGGGTKLEVK*
>SEQ ID NO: 4: Linker (KK)
   SSADDAKKDAAKKDDAKKDDAKKDAS
>SEQ ID NO: 5: Linker (AA)
   **SSADDAAADAAAADDAAADDAAADAS**
>SEQ ID NO: 6: mouse IgV signal sequence
   MKCSWIIFFLMAVVTGVNS
>SEQ ID NO: 7: notail deletion sequence
   PTNVSVSVIMSEGDGICY
>SEQ ID NO: 8: CHO W27 HAACOMP IgA H chain amino acid sequence (without signal sequence) 
EVQLQQSGSELVKSGASVKLSCTVSGFNFTDYYIHWVRQRTEQGLEWIGRIDPENDETTYAPKFQGK
ATMTADTSSNTAYLQLTSLTSEDTAVYYCARSTVLDYWGHGTTLTVSSESARNPTIYPLTLPRALSS
DPVIIGCLIHDYFPSGTMNVTWGKSGKDITTVNFPPALASGGGYTMSSQLTLPAVECPEGESVKCSV
QHDSNAVQELDVKCSGPPPPCPPCPPSCHPSLSLQRPALEDLLLGSDASLTCTLNGLRNPEGAVFTW
EPSTGKDAVQKKAVQNSCGCYSVSSVLPGCAERWNSGASFKCTVTHPESDTLTGTIAKITVNTFPPQ
VHLLPPPSEELALNELVSLTCLVRAFNPKEVLVRWLHGNEELSPESYLVFEPLKEPGEGATTYLVTS
VLRVSAELWKQGDQYSCMVGHEALPMNFTQKTIDRLSGKPTNVSVSVIMSEGDGICYSSADDAAADA
AAADDAAADDAAADASNSEGQIPLGGDLAPQMLRELQETNAALQDVRELLRQQVKEITFLKNTVMEC
DACGMQPARTPG*
>SEQ ID NO: 9: CHO W27 HKKCOMP IgA H chain amino acid sequence (without signal sequence) 
EVQLQQSGSELVKSGASVKLSCTVSGFNFTDYYIHWVRQRTEQGLEWIGRIDPENDETTYAPKFQGK
ATMTADTSSNTAYLQLTSLTSEDTAVYYCARSTVLDYWGHGTTLTVSSESARNPTIYPLTLPRALSS
DPVIIGCLIHDYFPSGTMNVTWGKSGKDITTVNFPPALASGGGYTMSSQLTLPAVECPEGESVKCSV
QHDSNAVQELDVKCSGPPPPCPPCPPSCHPSLSLQRPALEDLLLGSDASLTCTLNGLRNPEGAVFTW
EPSTGKDAVQKKAVQNSCGCYSVSSVLPGCAERWNSGASFKCTVTHPESDTLTGTIAKITVNTFPPQ
VHLLPPPSEELALNELVSLTCLVRAFNPKEVLVRWLHGNEELSPESYLVFEPLKEPGEGATTYLVTS
VLRVSAELWKQGDQYSCMVGHEALPMNFTQKTIDRLSGKPTNVSVSVIMSEGDGICYSSADDAKKDA
AAADDKKADDAKKDASNSEGQIPLGGDLAPQMLRELQETNAALQDVRELLRQQVKEITFLKNTVMEC
DACGMQPARTPG*
>SEQ ID NO: 10: CHO W27HdirectCOMP IgA H amino acid sequence (without signal sequence) 
EVQLQQSGSELVKSGASVKLSCTVSGFNFTDYYIHWVRQRTEQGLEWIGRIDPENDETTYAPKFQGK
ATMTADTSSNTAYLQLTSLTSEDTAVYYCARSTVLDYWGHGTTLTVSSESARNPTIYPLTLPRALSS
DPVIIGCLIHDYFPSGTMNVTWGKSGKDITTVNFPPALASGGGYTMSSQLTLPAVECPEGESVKCSV
QHDSNAVQELDVKCSGPPPPCPPCPPSCHPSLSLQRPALEDLLLGSDASLTCTLNGLRNPEGAVFTW
EPSTGKDAVQKKAVQNSCGCYSVSSVLPGCAERWNSGASFKCTVTHPESDTLTGTIAKITVNTFPPQ
VHLLPPPSEELALNELVSLTCLVRAFNPKEVLVRWLHGNEELSPESYLVFEPLKEPGEGATTYLVTS
VLRVSAELWKQGDQYSCMVGHEALPMNFTQKTIDRLSGKPTNVSVSVIMSEGDGICYNSEGQIPLGG
DLAPQMLRELQETNAALQDVRELLRQQVKEITFLKNTVMECDACGMQPARTPG*
>SEQ ID NO: 11: Base sequence of CHO W27HAACOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACTCCTCCGCTGACGATGCCGCTGCTGATGCTGCCGCCGCCG
ATGATGCTGCTGCTGACGACGCCGCTGCTGACGCTTCCAACTCCGAGGGCCAGATCCCGCTGGGTGG
AGACCTGGCCCCACAGATGCTGCGAGAACTTCAGGAGACTAATGCGGCGCTGCAAGACGTGAGAGAG
CTGTTGCGACAGCAGGTCAAGGAGATCACCTTCCTGAAGAATACGGTGATGGAATGTGATGCTTGCG
GAATGCAGCCCGCACGCACTCCAGGCTGA
>SEQ ID NO: 12: Base sequence of base sequence of CHO W27HKKCOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACAGCTCGGCAGACGATGCCAAGAAAGATGCAGCCAAGAAAG
ACGATGCCAAGAAAGACGACGCCAAGAAAGACGCTTCCAATTCAGAGGGCCAGATCCCGCTGGGTGG
AGACCTGGCCCCACAGATGCTGCGAGAACTTCAGGAGACTAATGCGGCGCTGCAAGACGTGAGAGAG
CTGTTGCGACAGCAGGTCAAGGAGATCACCTTCCTGAAGAATACGGTGATGGAATGTGATGCTTGCG
GAATGCAGCCCGCACGCACTCCAGGCTGA
>SEQ ID NO: 13: Base sequence of base sequence of CHO W27HdirectCOMP (including N-terminal signal sequence) 
ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAACCCACCAATGTCAGCGTGTCTGTGATC
ATGTCAGAGGGAGATGGCATCTGCTACAATTCAGAGGGCCAGATCCCGCTGGGTGGAGACCTGGCCC
CACAGATGCTGCGAGAACTTCAGGAGACTAATGCGGCGCTGCAAGACGTGAGAGAGCTGTTGCGACA
GCAGGTCAAGGAGATCACCTTCCTGAAGAATACGGTGATGGAATGTGATGCTTGCGGAATGCAGCCC
GCACGCACTCCAGGCTGA
>SEQ ID NO: 14: Base sequence of base sequence of CHO W27HnotailCOMP (including N-terminal signal sequence)

ATGAAATGCAGCTGGATCATCTTCTTCCTGATGGCAGTGGTTACAGGGGTCAATTCAGAGGTTCAGC
TGCAGCAGTCTGGGTCAGAGCTTGTGAAGTCTGGGGCCTCAGTCAAGTTGTCCTGCACAGTTTCTGG
GTTCAACTTTACAGACTACTATATACACTGGGTGAGGCAGAGGACTGAACAGGGCCTGGAATGGATT
GGAAGGATTGATCCTGAGAATGATGAAACTACATATGCCCCGAAATTCCAGGGCAAGGCCACTATGA
CAGCAGACACATCTTCCAACACAGCCTACCTGCAGCTCACCAGCCTGACATCTGAAGACACTGCCGT
CTATTACTGTGCTAGATCTACGGTCCTTGACTACTGGGGCCACGGCACCACTCTCACAGTCTCCTCA
GAGTCTGCGAGAAATCCCACCATCTACCCACTGACACTCCCACGAGCTCTGTCAAGTGACCCAGTGA
TAATCGGCTGCCTGATTCACGATTACTTCCCTTCCGGCACGATGAATGTGACCTGGGGAAAGAGTGG
GAAGGATATAACCACCGTAAACTTCCCACCTGCCCTGGCCTCTGGGGGAGGGTACACCATGAGCAGC
CAGTTGACCCTGCCAGCTGTCGAGTGCCCAGAAGGAGAATCCGTGAAATGTTCCGTGCAACATGACT
CTAACGCCGTCCAAGAATTGGATGTGAAGTGCTCTGGTCCTCCTCCTTGTCCTCCTTGTCCTCC
TTCCTGCCATCCCAGCCTGTCACTGCAGCGGCCAGCTCTTGAGGACCTGCTCCTGGGTTCAGATGCC
AGCCTCACATGTACTCTGAATGGCCTGAGAAATCCTGAGGGAGCTGTCTTCACCTGGGAGCCCTCCA
CTGGGAAGGATGCAGTGCAGAAGAAAGCTGTGCAGAATTCCTGCGGCTGCTACAGTGTGTCCAGCGT
CCTGCCTGGCTGTGCTGAGCGCTGGAACAGTGGCGCATCATTCAAGTGCACAGTTACCCATCCTGAG
TCTGACACCTTAACTGGCACAATTGCCAAAATCACAGTGAACACCTTCCCACCCCAGGTCCACCTGC
TACCGCCGCCGTCGGAGGAGCTGGCCCTGAATGAGCTCGTGTCCCTGACATGCCTGGTGCGAGCTTT
CAACCCTAAAGAAGTGCTGGTGCGATGGCTGCATGGAAATGAGGAGCTGTCCCCAGAAAGCTACCTA
GTGTTTGAGCCCCTAAAGGAGCCAGGCGAGGGAGCCACCACCTACCTGGTGACAAGCGTGTTGCGTG
TATCAGCTGAACTCTGGAAACAGGGTGACCAGTACTCCTGCATGGTGGGCCACGAGGCCTTGCCCAT
GAACTTCACCCAGAAGACCATCGACCGTCTGTCGGGTAAAAATTCAGAGGGCCAGATCCCGCTGGGT
GGAGACCTGGCCCCACAGATGCTGCGAGAACTTCAGGAGACTAATGCGGCGCTGCAAGACGTGAGAG
AGCTGTTGCGACAGCAGGTCAAGGAGATCACCTTCCTGAAGAATACGGTGATGGAATGTGATGCTTG
CGGAATGCAGCCCGCACGCACTCCAGGCTGA

## Claims

1. A method for producing an immunoglobulin multimer, the method comprising:
a step of bringing two or more immunoglobulins into contact with each other, the two or more immunoglobulins containing at least one immunoglobulin variant having a deletion in a C-terminal multimerization-promoting region of at least one heavy chain; and
a step of binding the two or more immunoglobulins.

2. The method according to claim 1, wherein the deletion is a deletion of one or more amino acids in the multimerization-promoting region.

3. The method according to claim 1, wherein a heterologous amino acid sequence that promotes multimerization is added to a C-terminus of an immunoglobulin having a deletion in a C-terminal multimerization-promoting region.

4. The method according to claim 1, wherein each of the plurality of immunoglobulin molecules is IgA or IgM.

5. An immunoglobulin multimer produced by the method according to claim 1.

6. An immunoglobulin multimer comprising two or more immunoglobulins,
wherein at least one of the immunoglobulins constituting the two or more immunoglobulins is an immunoglobulin variant having a deletion in a C-terminal multimerization-promoting region of at least one heavy chain.

7. The immunoglobulin multimer according to claim 5 or 6, wherein the immunoglobulin multimer has a bacterial growth inhibitory activity.

8. The immunoglobulin multimer according to claim 5 or 6, wherein the immunoglobulin multimer has a bacterial agglutination activity.

9. An immunoglobulin variant having a deletion in a C-terminal multimerization-promoting region and having an ability to form an immunoglobulin multimer.

10. A food composition, a feed composition, a pharmaceutical composition, or a reagent composition comprising the immunoglobulin multimer according to claim 5 or 6 or the immunoglobulin variant according to claim 9.

11. A nucleic acid comprising a nucleic acid sequence encoding the immunoglobulin variant according to claim 9.

12. A cell comprising the nucleic acid according to claim 11.
